# EUROPEAN PATENT APPLICATION

(11) **EP 2 020 214 A1**
(43) Date of publication of application: **04.02.2009**
(21) Application number: 08161475.2
(22) Date of filing: 30.07.2008
(51) Int. Cl.: A61F 9/008, G02B 6/38

(54) **Eye surgical unit and eye surgical instrument**

(30) Priority: 30.07.2007 NL 1034206; 12.09.2007 US 971641 P
(71) Applicant: D.O.R.C. Dutch Ophthalmic Research Center (International) B.V., 3214 VN Zuidland (NL)
(72) Inventor: Vijfvinkel, Gerrit Jan, 3211 BA, Geervliet (NL); Bätscher, Paul, CH-3714, Frutigen (CH); Widmer, Markus, DH-3600, Thun (CH)
(74) Representative: Hatzmann, Martin

(57) **Abstract**

The invention relates to an eye surgical unit, comprising a connecting module to align, upon coupling of the connecting module to a connecting module of an eye surgical instrument, an end of a light guide which is included in the connecting module of the eye surgical instrument with respect to the connecting module of the eye surgical unit. During coupling, the connecting modules of the eye surgical unit and the eye surgical instrument mutually cooperate. The mutual cooperation of the connecting modules is realized through a multiple-thread screw connection of which corresponding screw connection elements, upon coupling of the connecting modules through mutual rotation of the connecting modules, mutually engage substantially simultaneously.

## Description

The invention relates to an eye surgical unit, comprising a connecting module to align, upon coupling of the connecting module to a connecting module of an eye surgical instrument, an end of a light guide which is included in the connecting module of the eye surgical instrument with respect to the connecting module of the eye surgical unit, wherein during coupling the connecting modules of the eye surgical unit and the eye surgical instrument mutually cooperate.

Such eye surgical units are known, for instance for coupling eye surgical instruments to a laser unit, so that with the aid of the radiation generated by the laser unit, eye surgical operations, for instance photocoagulation, can be performed. In addition, the eye surgical instrument can comprise extra mechanical aids at a distal end of the instrument, such as a knife, scissors or tongs.

The light guide of the eye surgical instrument is aligned with respect to the connecting module of the eye surgical unit through coupling of the two connecting modules. Thus, a significant part of the light generated in the eye surgical unit can couple into the light guide of the eye surgical instrument.

When coupling the surgical unit and the surgical instrument, the connecting modules mutually cooperate. In known eye surgical units, the coupling is realized through a so-called SMA connection. By the use of an SMA connection, a reliable coupling has been obtained, provided that during coupling the connecting modules make a plurality of turns, for instance five, relative to each other so as to obtain a sufficiently solid connection that does not easily break loose, for instance when some tensile force is exerted on the coupling. It goes without saying that breaking loose of the coupling during use of the eye surgical unit is most undesirable. However, coupling the connecting modules by rotating them relative to each other a plurality of times requires a due amount of time and due discipline. When during routine operations rotation is not sufficiently sustained, still an unreliable coupling may thus be realized which cannot be recognized as such at first sight. Evidently, this is undesirable. For that matter, it also takes a due amount of time to uncouple the connecting modules by rotating the modules back relative to each other a plurality of times.

Furthermore, eye surgical units are known where the coupling between the connecting modules is realized through a bayonet closure. Rotation with a single turn of one module relative to the other module is then sufficient to realize the closure. However, a bayonet closure may also uncouple relatively easily.

The invention contemplates an eye surgical unit of the type described in the opening paragraph which, while preserving the advantages, obviates the disadvantages mentioned. In particular, the invention contemplates obtaining an eye surgical unit which allows a safer coupling between the eye surgical unit and an eye surgical instrument to be obtained. To that end, the mutual cooperation of the connecting modules is realized through a multiple-thread screw connection of which corresponding screw connection elements, upon coupling of the connecting modules through mutual rotation of the connecting modules, mutually engage substantially simultaneously.

By the use of a multiple-thread screw connection, a coupling has been obtained which enables a reliable connection since the coupling force is realized by the plurality of mutually engaging faces of the multiple-thread screw connection. By furthermore arranging for corresponding screw connection elements of the screw connections, upon coupling of the connecting modules through mutual rotation of the connecting modules, to mutually engage substantially simultaneously, a relatively limited rotation of the connecting modules can still yield a reliable coupling, since the corresponding screw connection elements, upon the rotation mentioned, all realize a screw connection with a certain coupling force. As the coupling between surgical unit and surgical instrument is reliable and can be realized fast, a safer coupling is obtained. Moreover, the coupling can also be removed faster.

It is noted that a light guide is understood to be a structure for guiding light, such as an optical fiber, also called fiber.

The invention also relates to an eye surgical instrument.

Further advantageous embodiments of the invention are represented in the subclaims.

The invention will be further elucidated on the basis of an exemplary embodiment which is represented in the drawing. In the drawing:
Figure 1 shows a schematic elevation of a partly cutaway eye surgical unit according to the invention;
Figure 2 shows a schematic elevation of a connecting module of the eye surgical unit of Figure 1 and a connecting module of an eye surgical instrument;
Figure 3 shows a schematic perspective elevation of a connecting module of the eye surgical unit of Figure 1; and
Figure 4 shows a schematic elevation of a partly cutaway connecting module of an eye surgical instrument according to the invention.

The Figures are only a schematic representation of a preferred embodiment of the invention. In the Figures, equal or corresponding parts are indicated with the same reference numerals.

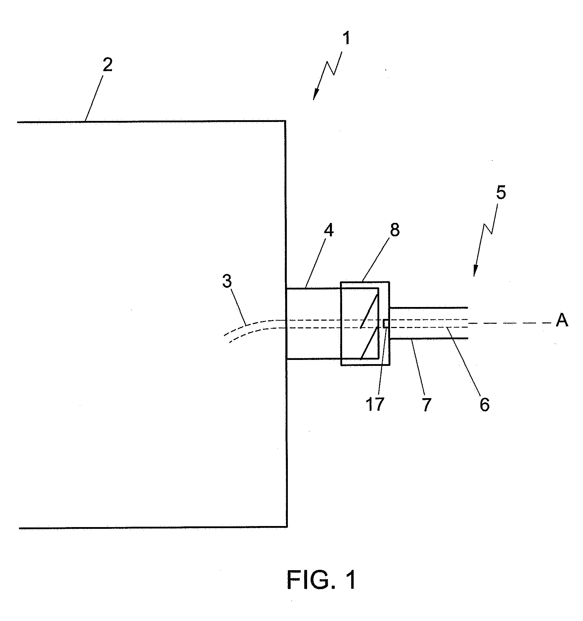

Figure 1 shows a schematic elevation of a partly cutaway eye surgical unit 1 according to the invention. The eye surgical unit 1 has a housing 2 in which a laser source, not shown, is arranged for generating one or more laser beams for eye surgical purposes such as photocoagulation. Furthermore, the eye surgical unit 1 has a light guide 3, also called fiber, and a connecting module 4 which is mounted on the outside of the housing 2. An end of the fiber 3 is included in the connecting module 4. During use of the surgical unit 1, a laser beam generated by the laser source is launched into the fiber 3 and propagates to the end that is included in the connecting module 4.

Figure 1 also shows a part of an eye surgical instrument 5, viz. another light guide 6, a sheathing 7 provided around the light guide, and another connecting module 8 in which an end of the light guide 6 of the eye surgical instrument 5 is included. Another end of the light guide 6 is included, via the sheathing 7, which forms a cord, in an eye surgical instrument module by means of which the surgeon performs the above-described operations. In addition, the eye surgical instrument module may optionally comprise extra components, such as a knife, scissors or tongs.

The connecting modules 4, 8 of the eye surgical unit 1 and the eye surgical instrument 5 can be coupled to each other as shown in Figure 1. The connecting modules 4, 8 mutually cooperate then. The cooperation takes place in that a multiple-thread screw connection takes care of the coupling. When coupling the connecting modules 4, 8 through mutual rotation of the connecting modules 4, 8, screw connection elements of the multiple-thread screw connection mutually engage substantially simultaneously. The rotation takes place about the common rotation axis A of the connecting modules 4, 8. By rotating one connecting module about the other, the coupling is thus obtained.

After coupling of the connecting modules 4, 8, the end 17 of the light guide 6 of the surgical instrument 5 is aligned with respect to the connecting module 4 of the eye surgical unit 1, so that the two light guides 3, 6 are aligned with respect to each other, so that at least a part of the light exiting from the light guide 3 of the surgical unit 1 is launched into the light guide 6 of the surgical instrument 5 and is transmitted to the eye surgical instrument module. After performing eye surgical operations, the surgical instrument 5 can be uncoupled from the eye surgical unit 1 again by rotating the connecting modules 4, 8 relative to each other in opposite direction.

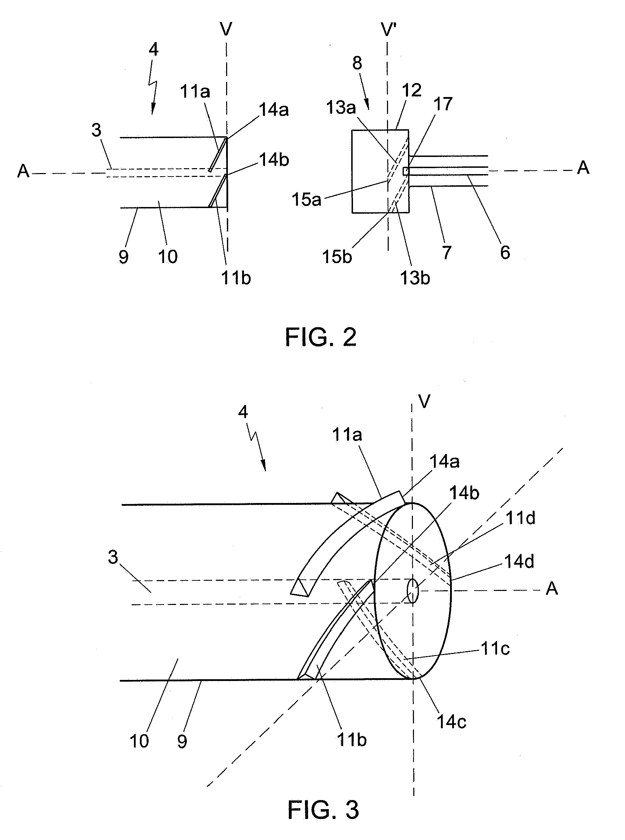

Figure 2 shows a schematic elevation of the connecting module 4 of the eye surgical unit 1 and the connecting module 8 of the eye surgical instrument 5 in uncoupled condition. The screw connection elements of the connecting module 4 of the eye surgical unit 1 comprise external thread 11 which is provided on the outer surface 9 of a cylinder 10 of circular cross section. Similarly, the connecting module 8 comprises a sleeve 12, with grooves 13 provided in the inner surface, corresponding to the external thread 11 of the connecting module 4 of the eye surgical unit 1. A thread 11 and a corresponding groove 13, in coupled condition, in pairs, form a screw connection. Thus, the plurality of threads 11a, 11b and the corresponding grooves 13a, 13b, in coupled condition, form a plurality of screw connections which form the multiple-thread screw connection. In coupled condition of the connecting modules 4, 8, the sleeve 12 extends at least partly over the cylinder 10.

It is noted that the grooves 13 in the sleeve may be formed by intermediate spaces between internal thread that may be provided in the inner surface of the sleeve 12.

Furthermore, it is noted that in another embodiment according to the invention, the connecting module 4 of the surgical unit 1 is provided with a sleeve with grooves and/or internal thread, while the connecting module of a surgical instrument 5 is provided with a cylinder with corresponding external thread.

Figure 3 shows a schematic perspective view of the connecting module 4 of the eye surgical unit 1. As shown in Figures 2 and 3, ends 14a, 14b, 14c, 14d of screw connection elements where, during mutual rotation of the connecting modules 4, 8, corresponding screw connection elements 11, 13 begin to engage each other, are situated substantially in the same plane V which is oriented transversely with respect to the common rotation axis A of the connecting modules 4, 8. Also the ends 15a, 15b of the grooves 13 which first engage the threads 11a, 11b are situated substantially in a same plane V' which is oriented transversely with respect to the common rotation axis A. In this way, when coupling the connecting modules 4, 8, the mutually corresponding screw connection elements mutually engage practically simultaneously. The screw connection elements are provided axially parallel with respect to the rotation axis A.

It is noted that the practically simultaneous mutual engagement can also be realized differently, for instance by arranging for corresponding ends of screw connection elements of a specific screw connection to be pairwise staggered over an equal distance axially with respect to the rotation axis A, such that upon coupling of the connecting modules 4, 8, still a practically simultaneous mutual engagement of screw connections is obtained.

The ends 14a, 14b, 14c, 14d of the screw connection elements where, during mutual rotation of the connecting module, corresponding screw connection elements begin to engage mutually, are distributed substantially proportionally in circumferential direction around the common rotation axis A of the connecting modules 4, 8. In this way, the coupling force of the screw connections is also distributed substantially proportionally in circumferential direction, which contributes to a stable coupling.

Furthermore, the number of screw connection elements, designed as external thread 11a, 11b, 11c, 11d, of the connecting module 4 of the eye surgical unit 1 is four. Naturally, also a different number of screw connection elements can be chosen, for instance two, three or more than four, for instance six, so that a corresponding number of screw connections are obtained for energization of the coupling between the connecting modules 4, 8.

In the embodiment shown, the screw connection elements are thus staggered through an angle of about 90 degrees in circumferential direction. Naturally, the screw connection elements may also be distributed differently, less proportionally in circumferential direction.

The screw elements 11a, 11b, 11c, 11d extend only over a part in circumferential direction, seen with respect to the common rotation axis A of the connecting modules. In the embodiment shown, the screw elements extend over a segment of about 90 degrees. However, the elements may also extend over a larger segment, for instance about 135 degrees or about 180 degrees or an angular segment therebetween. For that matter, the screw elements can also extend over a still larger segment, for instance at most once around, seen with respect to the common rotation axis A of the connecting modules 4, 8 or further than once around, for instance about one and a half times or twice around. The screw elements may be of relatively short design because the coupling force is obtained by the multiple-thread screw connection. Similarly, the pitch of the screw connections can be chosen to be relatively great.

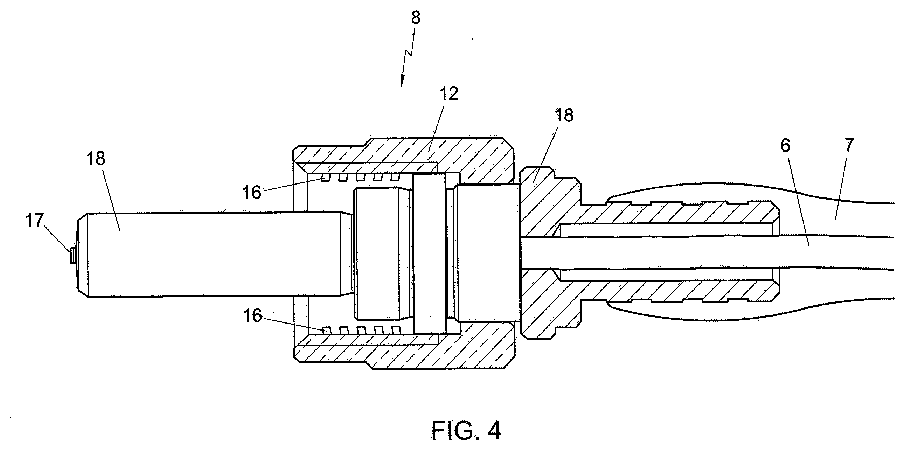

Figure 4 shows a schematic view of a partly cutaway different embodiment of a connecting module 8 of an eye surgical instrument 5 according to the invention. The connecting module 8 also comprises a sleeve 12 which can be coupled by means of a multiple-thread screw connection to the connecting module 4 of an eye surgical unit 1 according to the invention. To that end, the inner surface of the sleeve 12 is provided with multiple screw thread 16.

Furthermore, the connecting module 8 comprises a hollow cylindrical body 18 which encloses the end 17 of a light guide 6 included in the connecting module 8. After attachment of the connecting module 8 to the connecting module 4 of an eye surgical unit 1, the end 17 of the light guide 6 thus extends into the eye surgical unit 1. In an advantageous embodiment according to the invention, the eye surgical unit 1 does not comprise a light guide 3, but the connecting module 8 of the eye surgical instrument 5 is so dimensioned that the end 17 of the light guide 6 extends into the proximity of the laser source (not shown), so that optical losses are reduced, since the light generated by the laser source is then launched directly into the light guide of the eye surgical instrument 5. The shaft of the hollow cylindrical body 18 has an external diameter of about 2.9 mm. Naturally, different dimensioning is also possible.

In addition, the connecting module 8 comprises a coupling body 19 through which extends the light guide 6. Through the coupling body the light guide 6 is attached to the connecting module 8. The outer surface is provided with a relief, so that the sheathing 7 of the light guide 6 can be pulled over it. This prevents the sheathing 7 from rolling up and exposing the light guide 6 to external influences. It is noted that the light guide 6 and/or the sheathing 7 thereof may also be attached to the connecting module 8 otherwise, for instance with additional clamping elements.

The invention is not limited to the exemplary embodiments described here. Many variants are possible.

For instance, the connecting module 4 of the eye surgical unit 1 may be mounted not only on the outside of the housing 2, but also in a different manner, for instance wholly or partly in an opening of the housing 2 or on the inside of the housing 2, such that the connecting module 4 is accessible for the connecting module of an eye surgical instrument.

Such variants will be clear to those skilled in the art and are understood to fall within the scope of the invention as set forth in the following claims.

## Claims

1. An eye surgical unit, comprising a connecting module to align, upon coupling of the connecting module to a connecting module of an eye surgical instrument, an end of a light guide which is included in the connecting module of the eye surgical instrument with respect to the connecting module of the eye surgical unit, wherein during coupling the connecting modules of the eye surgical unit and the eye surgical instrument mutually cooperate, and wherein the mutual cooperation of the connecting modules is realized through a multiple-thread screw connection of which corresponding screw connection elements, upon coupling of the connecting modules through mutual rotation of the connecting modules, mutually engage substantially simultaneously.

2. An eye surgical unit according to claim 1, wherein ends of screw connection elements where, during mutual rotation of the connecting modules, corresponding screw connection elements begin to engage mutually, are situated substantially in the same plane which is oriented transversely with respect to the common rotation axis of the connecting modules.

3. An eye surgical unit according to claim 1 or 2, wherein ends of the screw connection elements where, during mutual rotation of the connecting modules, corresponding screw connection elements begin to engage mutually, are substantially proportionally distributed in circumferential direction around the common rotation axis of the connecting modules.

4. An eye surgical unit according to any one of the preceding claims, wherein a screw connection element of the connecting module of the eye surgical unit comprises internal or external thread.

5. An eye surgical unit according to any one of the preceding claims, wherein a screw connection element extends at most once around, viewed with respect to the common rotation axis of the connecting modules.

6. An eye surgical unit according to any one of the preceding claims, wherein a screw element extends only over a part in circumferential direction, viewed with respect to the common rotation axis of the connecting modules, preferably over a segment between about 90 degrees and about 180 degrees.

7. An eye surgical unit according to any one of the preceding claims, wherein the number of screw connection elements of the connecting module of the eye surgical unit is three or four.

8. An eye surgical instrument, comprising a light guide and a connecting module in which an end of the light guide is included in order to, upon coupling of the connecting module to a connecting module of an eye surgical instrument, align the light guide with respect to the connecting module of the eye surgical unit, wherein during coupling the connecting modules of the eye surgical unit and the eye surgical instrument mutually cooperate and wherein the mutual cooperation of the connecting modules is realized by a multiple-thread screw connection of which corresponding screw connection elements, upon coupling of the connecting modules through mutual rotation of the connecting modules, mutually engage substantially simultaneously.
